# EUROPEAN PATENT APPLICATION

(11) **EP 4 748 926 A1**
(43) Date of publication of application: **27.05.2026**
(21) Application number: 25790679.2
(22) Date of filing: 17.04.2025
(51) Int. Cl.: C12N 9/12, C12N 15/77, C12P 13/24, C12P 13/06, C12P 13/22

(54) **FRUCTOKINASE VARIANT AND METHOD FOR PRODUCING L-AMINO ACID USING SAME**

(30) Priority: 17.04.2024 KR 20240051536
(71) Applicant: CJ Cheiljedang Corporation, Seoul 04560 (KR)
(72) Inventor: YOON, Jeong-Hye, Seoul 04560 (KR); KIM, Kyungrim, Seoul 04560 (KR); KIM, Hee Ju, Seoul 04560 (KR); KIM, Seon Hye, Seoul 04560 (KR)
(74) Representative: Meissner Bolte Partnerschaft mbB
(86) International application number: PCT/KR2025/005231
(87) International publication number: WO 2025/221054

(57) **Abstract**

Provided are a fructokinase variant polypeptide; a polynucleotide encoding the variant polypeptide; a microorganism comprising the same; a method of producing an L-amino acid, the method comprising the step of culturing the microorganism in a medium; or use of the microorganism in producing an L-amino acid.

## Description

### [Technical Field]

The present disclosure relates to a fructokinase variant polypeptide; a polynucleotide encoding the variant polypeptide; a vector comprising the same; a microorganism comprising the polypeptide, polynucleotide, and/or vector; a method of producing an L-amino acid, the method comprising the step of culturing the microorganism in a medium; a composition for producing an L-amino acid, the composition comprising any one or more selected from the microorganism, a culture of the microorganism, and a fermentation product of the microorganism; or use of the microorganism in producing an L-amino acid.

### [Background Art]

Coryneform microorganisms are Gram-positive microorganisms that are frequently used in industrial production of substances with various applications such as feeds, pharmaceuticals, and foods comprising L-amino acids and various nucleic acids. In recent years, diamine, keto-acids, etc., have also been produced from coryneform microorganisms.

L-amino acids are basic structural units of proteins, and are used as important materials for pharmaceutical raw materials, food additives, animal feeds, nutritional supplements, pesticides, disinfectants, etc. Among L-amino acids, L-lysine is an essential amino acid that is not biosynthesized in the living body and is known to be necessary for growth promotion, calcium metabolism, promotion of gastric juice secretion, and resistance to diseases. L-lysine is widely used in feeds, medical products, foods, etc. Further, L-tryptophan is also one of the essential amino acids, and is used as a feed additive, an infusion solution, a raw material for pharmaceuticals, and a health food material, etc.

For production of amino acids, various studies have been conducted to develop microorganisms and fermentation process technologies for highly efficient production. For example, target-specific approaches have been mainly developed, such as a method of increasing expression of a gene encoding an enzyme involved in amino acid biosynthesis or a method of removing a gene unnecessary for amino acid biosynthesis in strains of the genus *Corynebacterium* (US 9109242 B2, US 8030036 B2). In addition to these methods, a method of removing a gene not involved in production of amino acids and a method of removing a gene whose specific functions are not known with regard to the production of amino acids have also been used. However, there is still a need for research into methods of efficiently producing L-amino acids with high yields.

### [Disclosure]

### [Technical Problem]

An object to be achieved in the present disclosure is to provide a microorganism comprising a fructokinase variant, and a method of producing an L-amino acid using the same.

### [Technical Solution]

One aspect of the present disclosure provides a fructokinase variant polypeptide, in which an amino acid corresponding to position 79 or position 181 of SEQ ID NO: 1 is substituted with a different amino acid.

In one specific embodiment, the variant polypeptide may have a substitution of the amino acid corresponding to position 79 of SEQ ID NO: 1 with cysteine or a substitution of the amino acid corresponding to position 181 of SEQ ID NO: 1 with valine, or a combination thereof.

In another specific embodiment, the variant polypeptide may consist of an amino acid sequence of SEQ ID NO: 3, SEQ ID NO: 5, or SEQ ID NO: 7.

In still another specific embodiment, the fructokinase may be encoded by *cscK* gene.

Another aspect of the present disclosure provides a polynucleotide encoding the variant polypeptide.

Still another aspect of the present disclosure provides a microorganism comprising any one or more selected from the variant polypeptide; the polynucleotide encoding the variant polypeptide; and a vector comprising the same.

With regard to the microorganism according to any one of the aforementioned specific embodiments, the microorganism may be a microorganism of the genus *Corynebacterium.*

With regard to the microorganism according to any one of the aforementioned specific embodiments, the microorganism of the genus *Corynebacterium* may be *Corynebacterium glutamicum.*

With regard to the microorganism according to any one of the aforementioned specific embodiments, the microorganism may have an increased ability to produce an L-amino acid, as compared to a microorganism comprising the polypeptide of SEQ ID NO: 1 or a polynucleotide encoding the same.

With regard to the microorganism according to any one of the aforementioned specific embodiments, the microorganism may have an increased ability to produce L-histidine, L-isoleucine, L-tryptophan, or L-lysine.

Still another aspect of the present disclosure provides a method of producing an L-amino acid, the method comprising the step of culturing the microorganism in a medium.

In one specific embodiment, the method may further comprise the step of recovering the L-amino acid from the cultured microorganism; a culture of the microorganism; a fermentation product of the microorganism; or the culture medium.

In another specific embodiment, the method may be a method of producing L-histidine, L-isoleucine, L-tryptophan, or L-lysine.

Still another aspect of the present disclosure provides a composition for producing an L-amino acid, the composition comprising any one or more selected from the variant polypeptide; the polynucleotide encoding the variant polypeptide; a vector comprising the same; a microorganism comprising the vector; a culture of the microorganism; and a fermentation product of the microorganism.

In one specific embodiment, the composition may be a composition for producing L-histidine, L-isoleucine, L-tryptophan, or L-lysine.

### [Advantageous Effects]

When a microorganism comprising a fructokinase variant polypeptide of the present disclosure is cultured, it is possible to produce L-amino acids with high yields, as compared to a microorganism having the existing unmodified polypeptide.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a fructokinase variant polypeptide, in which an amino acid corresponding to position 79 or position 181 of SEQ ID NO: 1 is substituted with a different amino acid.

As used herein, the term "fructokinase (CscK)" may refer to an enzyme that catalyzes the transfer of phosphate in the presence of ATP to produce D-fructose-6-phosphate and ADP.

With respect to the objects of the present disclosure, the protein may also be referred to as 'fructose phosphorylase', 'fructokinase', or 'CscK'. A gene encoding the protein may be, for example, *csck* gene, but is not limited thereto. In the present disclosure, the 'csck gene' may be used interchangeably with the 'fructokinase-encoding gene'. Furthermore, the protein may be derived from, for example, *Escherichia coli,* but is not particularly limited to its type as long as it possesses activity corresponding to fructokinase.

The fructokinase encoded by *csck* gene is known in the art, and the amino acid and polynucleotide sequences of the fructokinase may be obtained from a known database, for example, NCBI GenBank, etc., but are not limited thereto.

The fructokinase protein may comprise the amino acid sequence of SEQ ID NO: 1 or an amino acid sequence having 60% or more homology or identity thereto, but is not limited thereto as long as it has fructokinase activity. Specifically, addition of a meaningless sequence upstream or downstream of the amino acid sequence of SEQ ID NO: 1, a naturally occurring mutation, or a silent mutation thereof is not excluded, and as long as a protein has activity identical or corresponding to that of the protein comprising the amino acid sequence of SEQ ID NO: 1, it may belong to the protein which is the subject of the introduction of the mutation of the present disclosure. For example, the protein which is the subject of the introduction of the mutation of the present disclosure may be a protein consisting of the amino acid sequence of SEQ ID NO: 1 or an amino acid sequence having 60%, 70%, 75%, 80%, 85%, 90%, 95%, 96%, 97%, 98%, or 99% or more homology or identity thereto. Further, it is obvious that any variant having an amino acid sequence with deletion, modification, substitution, conservative substitution, or addition in part of the sequence may also fall within the scope of the polypeptide which is the subject of the introduction of the mutation of the present disclosure, as long as it is an amino acid sequence having such a homology or identity and exhibiting efficacy corresponding to that of the fructokinase.

In the amino acid sequence before modification of the fructokinase that is the subject of the mutation of the present disclosure, i.e., the sequence to be the parent sequence, the amino acids before modification corresponding to positions 79 and 181 of SEQ ID NO: 1 are tryptophan (W) and alanine(A), respectively.

As used herein, the term "variant polypeptide", "variant protein" or "variant" refers to a polypeptide in which one or more amino acids are conservatively substituted and/or modified so as to be different from the amino acid sequence of the variant before modification, while retaining functions or properties. Such a variant may generally be identified by modifying one or more amino acids in the amino acid sequence of the polypeptide and evaluating the properties of the modified polypeptide. In other words, the ability of the variant may be increased, unchanged, or decreased, as compared to the polypeptide before modification. Further, some variants may comprise variants in which one or more portions, such as an N-terminal leader sequence or transmembrane domain, have been removed. Other variants may comprise variants in which a portion has been removed from the N- and/or C-terminus of a mature protein. The term "variant" may also be used interchangeably with modification, modified polypeptide, modified protein, mutant, mutein, divergent, etc., and any term is not limited thereto, as long as it is used in the sense of being mutated.

The variant may also comprise deletion or addition of amino acids that have minimal influence on the properties and secondary structure of the polypeptide. For example, the polypeptide may be conjugated with an N-terminal signal (or leader) sequence of a protein involved in the transfer of proteins co-translationally or post-translationally. Further, the polypeptides may also be conjugated with another sequence or linker to identify, purify, or synthesize the polypeptides.

The fructokinase variant polypeptide of the present disclosure means a variant polypeptide in which the amino acid corresponding to position 79 or 181 from the N-terminus of SEQ ID NO: 1 is substituted with another amino acid.

The "fructokinase variant polypeptide" may be referred to as "fructokinase variant", "variant CscK", "CscK variant", etc. The fructokinase polypeptide of the present disclosure, which is a subject of the introduction of the mutation, may be used interchangeably with "CscK", but is not particularly limited thereto, encoded by *cscK* gene, and *cscK* derived from *Escherichia coli,* but is not limited thereto.

With regard to the fructokinase variant polypeptide of the present disclosure, the amino acid corresponding to position 79 or 181 in the amino acid sequence of SEQ ID NO: 1 may be substituted with an amino acid which is different from the amino acid before substitution.

In one specific embodiment, the variant polypeptide may have a substitution of the amino acid corresponding to position 79 of the amino acid sequence of SEQ ID NO: 1 with an amino acid other than tryptophan, which is the amino acid before substitution, and a substitution of the amino acid corresponding to position 181 of the amino acid sequence of SEQ ID NO: 1 with an amino acid other than alanine, which is the amino acid before substitution, or a combination thereof.

In another specific embodiment, with regard to the variant polypeptide, the amino acid corresponding to position 79 of SEQ ID NO: 1 may be substituted with an amino acid selected from the group consisting of asparagine, valine, glycine, leucine, arginine, alanine, methionine, threonine, glutamine, proline, isoleucine, serine, phenylalanine, histidine, cysteine, tyrosine, lysine, aspartate, and glutamic acid, but is not limited thereto.

In still another specific embodiment, with regard to the variant polypeptide, the amino acid corresponding to position 181 of SEQ ID NO: 1 may be substituted with an amino acid selected from the group consisting of asparagine, valine, glycine, leucine, arginine, tryptophan, methionine, threonine, glutamine, proline, isoleucine, serine, phenylalanine, histidine, cysteine, tyrosine, lysine, aspartate, and glutamic acid, but is not limited thereto.

In any one embodiment of the aforementioned embodiments, the variant polypeptide provided in the present disclosure may have a substitution of the amino acid corresponding to position 79 from the N-terminus of SEQ ID NO: 1 with cysteine, or a substitution of the amino acid corresponding to position 181 from the N-terminus of SEQ ID NO: 1 with valine, or a combination thereof.

In another embodiment of the aforementioned embodiments, the variant polypeptide provided in the present disclosure may comprise an amino acid sequence having at least 60%, 70%, 75%, 80%, 85%, 90%, 95%, 96%, 97%, 98%, 99%, 99.5% or 99.8% or more, or less than 100% homology or identity to SEQ ID NO: 1.

For example, the variant polypeptide of the present disclosure may comprise an amino acid sequence having at least 60%, 70%, 75%, 80%, 85%, 90%, 95%, 96%, 97%, 98%, 99%, 99.5%, or 99.8% or more homology or identity to SEQ ID NO: 1 while the amino acid corresponding to position 79 of the amino acid sequence represented by SEQ ID NO: 1 is fixed to cysteine. Further, the variant polypeptide of the present disclosure may comprise an amino acid sequence having at least 60%, 70%, 75%, 80%, 85%, 90%, 95%, 96%, 97%, 98%, 99%, 99.5%, or 99.8% or more homology or identity to SEQ ID NO: 1 while the amino acid corresponding to position 181 of the amino acid sequence represented by SEQ ID NO: 1 is fixed to valine. Further, the variant polypeptide of the present disclosure may comprise an amino acid sequence having at least 60%, 70%, 75%, 80%, 85%, 90%, 95%, 96%, 97%, 98%, 99%, 99.5%, or 99.8% or more homology or identity to SEQ ID NO: 1 while the amino acid corresponding to position 79 of the amino acid sequence represented by SEQ ID NO: 1 is fixed to cysteine, and the amino acid corresponding to position 181 is fixed to valine. Further, it is apparent that any variant polypeptide having an amino acid sequence with deletion, modification, substitution, conservative substitution, or addition in part of the sequence may also fall within the scope of the present disclosure, as long as it is an amino acid sequence having such a homology or identity and exhibiting efficacy corresponding to that of the variant polypeptide of the present disclosure.

Meanwhile, those skilled in the art may identify an amino acid which corresponds to position 79 or 181 of the amino acid sequence of SEQ ID NO: 1 of the present disclosure, in an arbitrary amino acid sequence through sequence alignment known in the art. Unless otherwise described in the present disclosure, when "an amino acid at a specific position of a specific sequence number" is described, it is apparent that the amino acid also comprises "an amino acid at the corresponding position" in an arbitrary amino acid sequence.

In still another specific embodiment, the variant polypeptide may consist of an amino acid sequence of SEQ ID NO: 3, SEQ ID NO: 5, or SEQ ID NO: 7.

Specifically, the variant polypeptide of the present disclosure may have or comprise the amino acid sequence of SEQ ID NO: 3, SEQ ID NO: 5, or SEQ ID NO: 7, or an amino acid sequence having at least 60%, 70%, 80%, 85%, 90%, 95%, 96%, 97%, 98%, or 99% or more homology or identity to the amino acid sequence of SEQ ID NO: 3, 5, or 7, or may consist of the amino acid sequence, or may essentially consist of the amino acid sequence.

Examples may comprise the case of having additions or deletions of sequences, which do not alter the function of the variant of the present disclosure, at the N-terminus, C-terminus, and/or inside of the amino acid sequence, a naturally occurring mutation, a silent mutation, or a conservative substitution.

The "different amino acid" is not limited as long as it differs from the amino acid before substitution. Meanwhile, in the present disclosure, when expressed as 'a specific amino acid is substituted', it is obvious that the amino acid is substituted with an amino acid different from the amino acid before substitution, even though it is not separately indicated that the amino acid is substituted with a different amino acid.

The amino acid may be generally classified, based on similarity in polarity, electric charge, solubility, hydrophobicity, hydrophilicity, and/or amphipathic nature of residues.

Examples of this classification may comprise positively charged (basic) amino acids such as arginine, lysine, and histidine; negatively charged (acidic) amino acids such as glutamic acid and aspartic acid; amino acids with nonpolar side chains (nonpolar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, and tyrosine. However, they are not necessarily limited thereto.

For example, when it is described that "the amino acid corresponding to position 79 of SEQ ID NO: 1 is substituted with a different amino acid", it may mean that the amino acid is substituted with asparagine, valine, glycine, alanine, glutamic acid (glutamate), phenylalanine, arginine, aspartate, cysteine, glutamine, histidine, proline, isoleucine, tyrosine, lysine, serine, methionine, threonine, or leucine, excluding tryptophan, but is not limited thereto.

For another example, when it is described that "the amino acid corresponding to position 181 of SEQ ID NO: 1 is substituted with a different amino acid", it may mean that the amino acid is substituted with asparagine, valine, glycine, tryptophan, glutamic acid (glutamate), phenylalanine, arginine, aspartate, cysteine, glutamine, histidine, proline, isoleucine, tyrosine, lysine, serine, methionine, threonine, or leucine, excluding alanine, but is not limited thereto.

For another example, when it is described that "the amino acids corresponding to positions 79 and 181 of SEQ ID NO: 1 are substituted with different amino acids", it may mean that the amino acid corresponding to position 79 is substituted with an amino acid other than tryptophan, and the amino acid corresponding to position 181 is substituted with an amino acid other than alanine, but is not limited thereto.

As used herein, although the expression "protein having an amino acid sequence described by a specific sequence number" is used, it is obvious that any protein having an amino acid sequence comprising deletion, modification, substitution, conservative substitution, or addition of some sequence may also be used in the present disclosure as long as the protein has activity identical or equivalent to that of the protein consisting of the amino acid sequence of the corresponding sequence number. Examples thereof do not exclude addition of a sequence, which does not alter the function of the protein, upstream or downstream of the amino acid sequence, a mutation that may occur naturally, a silent mutation thereof, or a conservative substitution, as long as it has activity identical or corresponding to that of the variant protein, and it would be obvious that even a protein with such a sequence addition or mutation falls within the scope of the present disclosure.

The "position N" of the present disclosure may comprise the position N and an amino acid position corresponding to the position N. Specifically, the position N may comprise an amino acid position corresponding to an arbitrary amino acid residue in a mature polypeptide, disclosed in a specific amino acid sequence. The specific amino acid sequence may be the amino acid sequence of SEQ ID NO: 1.

As used herein, the term "corresponding to" refers to an amino acid residue at a position listed in a polypeptide, or an amino acid residue similar to, identical to, or homologous to a residue listed in a polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 1, and based on this, each amino acid residue of the amino acid sequence may be numbered with reference to the numerical position of the amino acid residue corresponding to the amino acid residue of SEQ ID NO: 1. For example, a sequence alignment algorithm as described in the present disclosure may determine the position of an amino acid, or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but are not limited thereto, and a sequence alignment program, a pairwise sequence comparison algorithm, etc., which known in the art, may be appropriately used.

As used herein, the term "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. For example, positively charged (basic) amino acids comprise arginine, lysine, and histidine; negatively charged (acidic) amino acids comprise glutamic acid and aspartate; aromatic amino acids comprise phenylalanine, tryptophan, and tyrosine; and hydrophobic amino acids comprise alanine, valine, isoleucine, leucine, methionine, phenylalanine, tyrosine, and tryptophan. Further, amino acids may also be classified into amino acids with electrically charged side chains and amino acids with uncharged side chains. The amino acids with electrically charged side chains comprise aspartic acid, glutamic acid, lysine, arginine, and histidine, and the amino acids with uncharged side chains may be classified again into nonpolar amino acids and polar amino acids. The nonpolar amino acids comprise glycine, alanine, valine, leucine, isoleucine, methionine, phenylalanine, tryptophan, and proline, and the polar amino acids comprise serine, threonine, cysteine, tyrosine, asparagine, and glutamine. Usually, conservative substitution may hardly affect or not affect the activity of produced polypeptides. Usually, conservative substitution may hardly affect or may not affect the activity of proteins or polypeptides.

Still another aspect of the present disclosure provides a polynucleotide encoding the variant polypeptide.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant.

The polynucleotide encoding the fructokinase variant polypeptide of the present disclosure may comprise any polynucleotide sequence without limitation as long as it is a polynucleotide sequence encoding the variant polypeptide having fructokinase activity.

For example, the polynucleotide encoding the fructokinase variant polypeptide of the present disclosure may be a polynucleotide sequence encoding the amino acid sequence of the fructokinase variant polypeptide of the present disclosure, but is not limited thereto.

For example, the polynucleotide of the present disclosure may comprise a nucleotide sequence encoding the amino acid sequence represented by SEQ ID NO: 3, SEQ ID NO: 5, or SEQ ID NO: 7. For example, the polynucleotide of the present disclosure may have or comprise SEQ ID NO: 4, SEQ ID NO: 6, or SEQ ID NO: 8. Further, the polynucleotide of the present disclosure may consist of or essentially consist of the sequence of SEQ ID NO: 4, SEQ ID NO: 6, or SEQ ID NO: 8.

The polynucleotide of the present disclosure may undergo various modifications in the coding region within the scope that does not change the amino acid sequence of the variant of the present disclosure in consideration of codon degeneracy or codons preferred in organisms that are intended to express the variant of the present disclosure. Therefore, it is obvious that, due to codon degeneracy, a polynucleotide to be translated into a polypeptide consisting of the amino acid sequence of the variant of the present disclosure or a polypeptide having homology or identity thereto may also be comprised. For example, the polynucleotide of the present disclosure may be the sequence of SEQ ID NO: 4, SEQ ID NO: 6, or SEQ ID NO: 8; or a degenerate sequence thereof.

For example, the polynucleotide of the present disclosure may comprise substitution of codons encoding tryptophan, which are amino acids at positions 235 to 237 of SEQ ID NO: 2, with codons encoding an amino acid other than tryptophan, e.g., cysteine; substitution of codons encoding alanine, which are amino acids corresponding to positions 541 to 543, with codons encoding an amino acid other than alanine, e.g., valine in a nucleotide sequence having 60%, 70%, 75%, 80%, 85%, 90%, 95%, 96%, 97%, 98%, 99%, 99.5%, 99.7%, or 99.8% or more homology or identity to the sequence of SEQ ID NO: 2; or both thereof, but is not limited thereto.

It is also apparent that a variant having a polynucleotide sequence having deletion, modification, substitution, conservative substitution, or addition of some sequence also falls within the scope of the present disclosure as long as the polynucleotide sequence has such a homology or identity and encodes the amino acid sequence of the fructokinase variant polypeptide of the present disclosure.

For another example, the polynucleotide of the present disclosure may have or comprise a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 4, SEQ ID NO: 6, SEQ ID NO: 8, or may consist of or may essentially consist of a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 4, SEQ ID NO: 6, SEQ ID NO: 8, but is not limited thereto.

Further, the polynucleotide of the present disclosure may comprise a probe that may be prepared from a known gene sequence; for example, it may comprise any sequence without limitation as long as it is a sequence that is able to hybridize with a complementary sequence to the entirety or a part of the polynucleotide sequence of the present disclosure under stringent conditions.

The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof comprise a condition in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "homology" or "identity" means the relevance between two given amino acid sequences or nucleotide sequences and may be expressed as a percentage. The terms "homology and identity" may often be used interchangeably.

The sequence homology or identity of conserved polynucleotides or polypeptides is determined by standard alignment algorithms, and a default gap penalty established by the program being used may be used together. Substantially, homologous or identical sequences may generally hybridize with the entirety or a part of the sequence under moderately or highly stringent conditions. It is apparent that hybridization also comprises hybridization of a polynucleotide with a polynucleotide comprising a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387(1984)), BLASTP, BLASTN1, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST or ClustalW of the National Center for Biotechnology Information may be used to determine the homology, similarity, or identity.

The homology, similarity, or identity of polynucleotides or polypeptides may be, for example, determined by comparing sequence information using, for example, the GAP computer program, such as Needleman et al.(1970), J Mol Biol. 48:443, as disclosed in Smith and Waterman, Adv. Appl. Math(1981) 2:482. Briefly, the GAP program defines the homology, similarity, or identity as the value obtained by dividing the number of similarly aligned symbols (i.e., nucleotides or amino acids) by the total number of symbols in the shorter of the two sequences. Default parameters for the GAP program may comprise (1) a binary comparison matrix (comprising a value of 1 for identities and 0 for non-identities) and the weighted comparison matrix of Gribskov et al. (1986) Nucl. Acids Res. 14: 6745, as disclosed in Schwartz and Dayhoff, eds., Atlas Of Protein Sequence And Structure, National Biomedical Research Foundation, pp. 353-358(1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" used herein represents the relevance between sequences.

Still another aspect of the present disclosure provides a vector comprising the polynucleotide of the present disclosure. The vector may be an expression vector for expressing the polynucleotide in a microorganism, but is not limited thereto.

As used herein, the term "vector" may comprise a DNA construct comprising a nucleotide sequence of a polynucleotide encoding a polypeptide of interest which is operably linked to a suitable expression regulatory region (or expression control sequence) so that the polypeptide of interest may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. Once transformed into a suitable microorganism, the vector may replicate or function independently from the host genome, or may integrate into the genome thereof.

The vector that may be used in the present disclosure is not particularly limited, and any vector known in the art may be used. Examples of vectors commonly used may comprise natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, etc. may be used, and as a plasmid vector, those based on pDZ, pBR, pUC, pBluescriptll, pGEM, pTZ, pCL and pET, etc. may be used. Specifically, pDZ, pDC, pDCM2, pDC24, pACYC177, pACYC184, pCL, pECCG117, pUC19, pBR322, pMW118, pCC1BAC vectors, etc. may be used.

For example, the polynucleotide encoding the target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further comprise a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cytotoxic agents, or expression of surface polypeptides, may be used. Only cells expressing the selection marker are able to survive or show a different phenotype under the environment treated with the selective agent, and thus the transformed cells may be selected.

As used herein, the term "transformation" means that a vector comprising a polynucleotide encoding a target polypeptide is introduced into a microorganism or a microorganism so that the polypeptide encoded by the polynucleotide may be expressed in the microorganism. The transformed polynucleotide may be located regardless of the position, either by being inserted into the chromosome of the microorganism or located outside the chromosome as long as it may be expressed in the microorganism. Further, the polynucleotide comprises DNA and/or RNA encoding the target polypeptide. The polynucleotide may be introduced in any form as long as it may be introduced into a microorganism and expressed. For example, the polynucleotide may be introduced into a microorganism in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a microorganism in its own form and operably linked to a sequence required for expression in the microorganism, but is not limited thereto.

Further, the term "operably linked" means that the polynucleotide sequence is functionally linked to a promoter sequence that initiates and mediates transcription of the polynucleotide encoding the target variant of the present disclosure.

The method of transforming the vector of the present disclosure comprises any method of introducing the nucleic acid into a cell, and may be performed by selecting a suitable standard technique known in the art according to the host cell. For example, the transformation method may comprise electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, etc., but is not limited thereto.

Still another aspect of the present disclosure provides a microorganism comprising any one or more selected from the variant polypeptide; the polynucleotide encoding the variant polypeptide; and a vector comprising the same.

In one specific embodiment, the microorganism of the present disclosure may be a microorganism having an ability to produce an L-amino acid.

As used herein, the term "microorganism (or strain)" comprises all wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism comprising genetic modification for production of a target polypeptide, protein, or product. As used herein, the terms "microorganism", "strain", and "microorganism" have the same meaning and may be used interchangeably without limitation.

As used herein, the term "recombinant microorganism" refers to a microorganism that has been genetically modified and exhibits a different genotype and/or phenotype compared to a naturally occurring microorganism (e.g., when the genetic modifications have an effect on a coding nucleic acid sequence of a microorganism), and may comprise progeny or all potential progeny of the microorganism. As used herein, the terms "recombinant microorganism", "genetically modified microorganism", "recombinant host cell", "recombinant cell", and "recombinant strain" may be used interchangeably. The recombinant microorganism, for example, may express genes that are not found in the native (non-recombinant) form; or may not express genes that are expressed in its native form; or may express native genes in a manner different from that in its native form.

As used herein, the term "microorganism producing an L-amino acid" refers to a prokaryotic or eukaryotic microbial strain capable of producing the L-amino acid within a living organism, and may comprise both a microorganism prepared by providing the ability to produce the L-amino acid to a parent strain without the ability to produce the L-amino acid, or a microorganism that inherently has the ability to produce the L-amino acid. The ability to produce the L-amino acid may be conferred or enhanced by species improvement.

As used herein, the term "unmodified microorganism" does not exclude strains comprising mutations that may occur naturally in microorganisms, and may be a wild-type strain or a natural strain itself or may be a strain before the trait is changed by genetic variation due to natural or artificial factors. For example, the unmodified microorganism may be a strain into which the fructokinase variant polypeptide described herein is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

The microorganism having the ability to produce an L-amino acid of the present disclosure may be a microorganism comprising any one or more of the variant polypeptide of the present disclosure, the polynucleotide of the present disclosure, and the vector comprising the polynucleotide of the present disclosure; a microorganism which is modified to express the variant polypeptide of the present disclosure or the polynucleotide of the present disclosure; a microorganism (e.g., recombinant strain) expressing the variant polypeptide of the present disclosure or the polynucleotide of the present disclosure; or a microorganism (e.g., recombinant strain) having the activity of the fructokinase variant polypeptide of the present disclosure, but is not limited thereto.

For example, the strain of the present disclosure is a cell or microorganism which is transformed with the vector comprising the polynucleotide encoding the variant polypeptide of the present disclosure to express the variant fructokinase comprising the variant polypeptide of the present disclosure, and the strain of the present disclosure may comprise any microorganism that is able to produce the L-amino acid by comprising the variant polypeptide of the present disclosure.

For example, the microorganism of the present disclosure may be a recombinant strain having the increased ability to produce the L-amino acid due to expression of the fructokinase variant polypeptide by introducing the polynucleotide encoding the variant polypeptide of the present disclosure into a natural wild-type microorganism or a microorganism having ability to produce the L-amino acid. The recombinant strain having the increased ability to produce the L-amino acid may be a microorganism in which the ability to produce the L-amino acid is increased, as compared to the natural wild-type microorganism or fructokinase unmodified microorganism (e.g., a microorganism expressing the wild-type fructokinase or a microorganism not expressing the variant polypeptide of the present disclosure), but is not limited thereto. For example, the microorganism having the increased ability to produce the L-amino acid of the present disclosure may be a microorganism in which the ability to produce the L-amino acid is increased, as compared to the microorganism comprising the polypeptide of SEQ ID NO: 1 or the polynucleotide encoding the same, but is not limited thereto. For example, the unmodified microorganism, which is the target strain being compared to determine whether or not the ability to produce the L-amino acid is increased, may be a *Corynebacterium glutamicum* strain, CA14-0114; KCCM12739P; CA04-8405; or KCCM11016P strain, but is not limited thereto.

The microorganism of the present disclosure may comprise any microorganism which is able to express the fructokinase variant polypeptide of the present disclosure by a variety of known methods in addition to introduction of the nucleotide or vector.

For example, the microorganism having the increased ability to produce the L-amino acid may have an increased ability to produce the L-amino acid of about 1% or more, specifically, about 1% or more, about 2% or more, about 3% or more, about 4% or more, about 5% or more, about 6% or more, about 7% or more, about 8% or more, about 9% or more, about 10% or more, about 11% or more, about 12% or more, about 13% or more, about 14% or more, about 15% or more, about 16% or more, about 17% or more, about 18% or more, about 19% or more, about 20% or more, about 25% or more, about 30% or more, about 35% or more, about 40% or more, about 50% or more (the upper limit is not particularly limited, but may be, for example, about 200% or less, about 150% or less, about 100% or less, about 90% or less, about 80% or less, about 70% or less, about 60% or less, about 55% or less, or about 50% or less), as compared to that of the parent strain before modification or unmodified microorganism, but the increased amount is not limited thereto as long as the production ability has an increased amount of a + value, as compared to the production ability of the parent strain before modification or the unmodified microorganism. In another example, the recombinant strain having the increased ability to produce the L-amino acid may have an increased ability to produce the L-amino acid of about 1.01 times or more, about 1.02 times or more, about 1.03 times or more, about 1.04 times or more, 1.05 times or more, 1.06 times or more, 1.07 times or more, 1.08 times or more, 1.09 times or more, about 1.1 times or more, about 1.11 times or more, about 1.12 times or more, about 1.13 times or more, about 1.14 times or more, about 1.15 times or more, about 1.16 times or more, about 1.17 times or more, about 1.18 times or more, about 1.19 times or more, about 1.20 times or more, about 1.25 times or more, about 1.30 times or more, about 1.35 times or more, about 1.40 times or more, about 1.45 times or more, or about 1.50 times or more (the upper limit is not particularly limited, but may be, for example, about 10 times or less, about 5 times or less, about 3 times or less, about 2 times or less, or about 1.5 times or less), as compared to that of the parent strain before modification or the unmodified microorganism, but is not limited thereto. As used herein, the term "about" refers to a range which comprises all of ±0.5, ±0.4, ±0.3, ±0.2, ±0.1, etc., and comprises all values that are equivalent or similar to those following the term "about", but the range is not limited thereto.

With regard to the microorganism according to any one of the aforementioned specific embodiments, the microorganism of the present disclosure may be a microorganism of the genus *Corynebacterium* (*Corynebacteria* sp.), the genus *Escherichia* (*Escherichia* sp.), the genus *Erwinia* (Erwinia sp.), the genus *Serratia* (*Serratia* sp.), the genus *Providencia* (*Providencia* sp.), the genus *Pseudomonas* (*Pseudomonas* sp.), the genus *Leptospira* (*Leptospira* sp.), the genus *Salmonella* (*Salmonella* sp.), the genus *Brevibacteria* (*Brevibacteria* sp.), the genus *Hypomononas* (*Hypomononas* sp.), the genus *Chromobacterium* (*Chromobacterium* sp.) and the genus *Norcardia* (*Norcardia* sp.), fungi, or yeasts, specifically, a microorganism of the genus *Corynebacterium,* but is not limited thereto.

For example, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the genus *Corynebacterium,* more specifically, *Corynebacterium glutamicum,* but is not limited thereto.

Specifically, the microorganism of the present disclosure may be a microorganism of the genus *Corynebacterium,* more specifically, *Corynebacterium glutamicum,* but is not limited thereto.

Meanwhile, the microorganism of the genus *Corynebacterium* of the present disclosure, in which the ability to produce the L-amino acid is improved, may comprise any of a natural wild-type microorganism itself, a microorganism of the genus *Corynebacterium* which has the improved ability to produce the L-amino acid by increasing or decreasing the activity of a gene related to the production mechanism of the L-amino acid, and a microorganism of the genus *Corynebacterium* which has the improved ability to produce the L-amino acid by introducing or increasing the activity of a foreign gene. In one specific embodiment, the microorganism may comprise a microorganism of the genus *Corynebacterium,* in which the ability to produce the L-amino acid is improved by increasing the fructokinase activity due to introduction of the variant polypeptide of the present disclosure, but is not limited thereto.

As used herein, the term "increase" of the protein (polypeptide) activity means that the activity of the protein (polypeptide) is increased in host cells (microorganisms), as compared to the endogenous activity thereof. The increase may be used interchangeably with terms such as activation, up-regulation, overexpression, and enhancement, etc. The host cells (microorganisms) may be prokaryotic or eukaryotic microorganisms.

The increase of the protein (polypeptide) activity may comprise both exhibiting the protein (polypeptide) activity that was not originally possessed by host cells (microorganisms) and exhibiting improved protein (polypeptide) activity as compared to the endogenous activity or activity before modification.

For example, the "exhibiting the protein (polypeptide) activity that was not originally possessed" or "exhibiting improved protein (polypeptide) activity" may be caused by "introducing a protein (polypeptide)," but is not limited thereto.

As used herein, the term "introducing a protein (polypeptide)" means that a gene not originally possessed by a microorganism is expressed within the microorganism, thereby exhibiting the activity of the specific protein, or exhibiting the enhanced, increased, or improved polypeptide activity, as compared to the endogenous activity or activity before modification of the corresponding protein. For example, it may be caused by introducing a gene encoding the protein (polypeptide) into the host cells (microorganisms). For example, a polynucleotide encoding a specific protein (polypeptide) may be introduced into a chromosome within host cells (microorganisms), or a vector comprising the polynucleotide encoding the specific protein (polypeptide) may be introduced into the host cells (microorganisms), thereby exhibiting or improving activity thereof.

The "endogenous activity" means the activity of a specific protein (polypeptide) originally possessed by a host cell (microorganism) before the trait is changed or by an unmodified host cell (microorganism), when the trait is changed by genetic variation due to natural or artificial factors. This may be used interchangeably with "activity before modification".

The fact that the activity of the protein (polypeptide) is increased as compared to the endogenous activity means that the activity and/or concentration (expression level) of the protein (polypeptide) of the host cell (microorganism) is improved as compared to the activity and/or concentration (expression level) of the protein (polypeptide) originally possessed by the host cell (microorganism) before the trait is changed or by the unmodified host cell (microorganism).

For example, the increase indicates that the activity of the corresponding protein (polypeptide) originally absent appears, or its activity or concentration is generally increased to about 1% or more, about 10% or more, about 25% or more, about 50% or more, about 75% or more, about 100% or more, about 150% or more, about 200% or more, about 300% or more, about 400% or more, or about 500% or more, up to about 1000% or about 2000% or more, based on the activity or concentration in the host cell (microorganism) before the trait is changed or the unmodified host cell (microorganism), but is not limited thereto.

The increase of the activity of the protein (polypeptide) may be achieved through the introduction of a foreign protein (polypeptide) or the increase of the activity of the endogenous protein (polypeptide). The increase of the activity of the protein (polypeptide) may be confirmed by enhancement in the degree of activity and the expression level of the corresponding protein (polypeptide) or by an increase in the amount of the product resulting from the activity of the corresponding protein (polypeptide).

The increase of the activity of the protein (polypeptide) may be achieved by various methods well known in the art, and the method is not limited as long as the activity of the target protein (polypeptide) may be increased as compared to that of the host cell (microorganism) before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the increase of the activity of the protein (polypeptide) of the present disclosure may be:
1) increase in the intracellular copy number of the polynucleotide encoding the protein (polypeptide);
2) modification of a gene expression regulatory region on a chromosome encoding the protein (polypeptide) (e.g., introduction of variations into the expression regulatory region, replacement with a sequence having stronger activity, or insertion of a sequence having stronger activity);
3) modification of a nucleotide sequence encoding an initiation codon or 5'-UTR region of the gene transcript encoding the protein (polypeptide);
4) modification of the amino acid sequence of the protein (polypeptide) to increase the activity of the protein (polypeptide);
5) modification of the polynucleotide sequence encoding the protein (polypeptide) to increase the activity of the protein (polypeptide) (e.g., modification of the polynucleotide sequence of the protein (polypeptide) coding gene to encode a protein (polypeptide) that has been modified to increase the activity of the protein (polypeptide));
6) introduction of a foreign protein (polypeptide) exhibiting the activity of the protein (polypeptide) or a foreign polynucleotide encoding the same;
7) codon optimization of the polynucleotide encoding the protein (polypeptide);
8) analysis of the tertiary structure of the protein (polypeptide) to select and modify or chemically modify the exposed site;
9) control of intracellular localization of the protein (polypeptide); or
10) a combination of two or more selected from 1) to 9), but is not particularly limited thereto.

For example,
1) the increase in the intracellular copy number of the polynucleotide encoding the protein (polypeptide) may be achieved by the introduction, into a host cell (microorganism), of a vector comprising the polynucleotide encoding the protein (polypeptide), which is operably linked to an appropriate regulatory sequence. Alternatively, the increase may be achieved by introducing one copy or two or more copies of the polynucleotide encoding the protein (polypeptide), which is operably linked to an appropriate regulatory sequence, into a chromosome of a host cell (microorganism). The introduction into the chromosome may be performed by the introduction, into the host cell (microorganism), of a vector capable of inserting the polynucleotide into the chromosome of the host cell (microorganism), but is not limited thereto. The vector is as described above. The regulatory sequence may be a native (derived from the same origin) or foreign (derived from different genes) sequence to the coding polynucleotide sequence, or a mutant sequence or other artificial sequence thereof, and may induce expression of the polynucleotide in the host cell (microorganism).
2) The replacement of a gene expression regulatory region (or expression regulatory sequence) on the chromosome encoding the protein (polypeptide) with a sequence with strong activity may be, for example, introduction of a mutation into the sequence through deletion, insertion, substitution, or a combination thereof, or replacement with a sequence having stronger activity, to further increase the activity of the expression regulatory region. The expression regulatory region may comprise, but is not particularly limited to, a promoter, an operator sequence, a sequence encoding a ribosome binding site, a sequence controlling the termination of transcription and translation, etc. For example, it may be the replacement of the original promoter with a stronger promoter, but is not limited thereto.

Examples of known stronger promoters may comprise cj1 to cj7 promoters (US Patent No. 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. 10584338 B2), O2 promoter (US Patent No. 10273491 B2), tkt promoter, yccA promoter, etc., but are not limited thereto.

3) The modification of a nucleotide sequence encoding an initiation codon or 5'-UTR region of the gene encoding the protein (polypeptide) may be, for example, replacing it with another initiation codon having a higher protein (polypeptide) expression rate than the endogenous initiation codon, or modifying it to encode a ribosome binding site (RBS) sequence having a higher protein (polypeptide) expression rate than the endogenous RBS sequence, but is not limited thereto.

4) and 5) The modification of the amino acid sequence or polynucleotide sequence of the protein (polypeptide) may be performed by introducing a mutation into the amino acid sequence of the protein (polypeptide) or the polynucleotide sequence encoding the protein (polypeptide) by deletion, insertion, substitution, or a combination thereof to increase the activity of the protein (polypeptide), or by replacing it with an amino acid sequence or a polynucleotide sequence which is modified to increase the activity, but is not limited thereto. The replacement may be performed by, for example, inserting the polynucleotide into the chromosome by homologous recombination, but is not limited thereto.

6) The introduction of a foreign polynucleotide exhibiting the activity of the protein (polypeptide) may be introduction, into a host cell (microorganism), of a foreign polynucleotide encoding a protein (polypeptide) exhibiting activity identical or similar to that of the protein (polypeptide). There is no limitation on its origin or sequence as long as the foreign polynucleotide exhibits activity identical or similar to that of the protein (polypeptide). The method used in the introduction may be performed by appropriately selecting a known transformation method by those skilled in the art. As the introduced polynucleotide is expressed in the host cell, the protein (polypeptide) may be produced and the activity thereof may be increased.

7) The codon optimization of the polynucleotide encoding the protein (polypeptide) may be codon optimization of an endogenous polynucleotide so as to enhance transcription or translation in a host cell (microorganism), or codon optimization of a foreign polynucleotide so as to perform optimized transcription and translation in a host cell (microorganism).

8) The analysis of the tertiary structure of the protein (polypeptide) to select and modify or chemically modify the exposed site may be, for example, to determine a template protein candidate according to the degree of similarity of the sequence by comparing the sequence information of a protein (polypeptide) to be analyzed with a database storing the sequence information of known proteins, to confirm the structure based on this, and to select and to modify or chemically modify the exposed site to be modified or chemically modified.

9) The control of intracellular localization of the protein (polypeptide) may be targeting the protein (polypeptide) to a specific organelle within cells or to a specific intracellular space. For example, it may be targeting to the periplasm or cytoplasm through the addition or removal of a leader sequence that functions in targeting the protein (polypeptide), but is not limited thereto.

Such an increase of the activity of the protein (polypeptide) may be an increase of the activity or concentration of the corresponding protein (polypeptide), based on the activity or concentration of the protein (polypeptide) expressed in a wild-type or a host cell (microorganism) before modification, or an increase in the amount of a product resulting from the activity of the corresponding protein (polypeptide), but is not limited thereto.

The modification of a part or the entirety of the polynucleotide in the microorganism of the present disclosure may be induced by: (a) homologous recombination using a vector for chromosomal insertion into the microorganism or genome editing using engineered nuclease (e.g., CRISPR-Cas9), and/or (b) treatment with light, such as ultraviolet light and radiation, and/or chemicals, but is not limited thereto.

The microorganism of the present disclosure may have an improved ability to produce an L-amino acid.

As used herein, the "L-amino acid" comprises all L-amino acids that may be produced by microorganisms through metabolic processes from various carbon sources, and specifically, may comprise, but is not limited to, L-histidine, L-isoleucine, L-tryptophan, or L-lysine.

In one specific embodiment of the present disclosure, the microorganism of the genus *Corynebacterium* may have an increased ability to produce any one or more amino acids selected from L-histidine, L-isoleucine, L-tryptophan, and L-lysine, as compared to an unmodified microorganism.

Still another aspect of the present disclosure provides a method of producing an L-amino acid, the method comprising the step of culturing, in a medium, the microorganism comprising the variant polypeptide of the present disclosure; the polynucleotide encoding the variant polypeptide; or the vector comprising the polynucleotide.

The microorganism is as described in other aspects.

As used herein, the term "culturing" means that the microorganism of the present disclosure is grown under appropriately controlled environmental conditions. In the present disclosure, the culturing process may be performed according to appropriate media or culture conditions known in the art. Such a culturing process may be easily adjusted for use by those skilled in the art according to the strain to be selected. Specifically, the culturing may be of a batch type, a continuous type, and/or a fed-batch type, but is not limited thereto.

The microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or decomposition products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These components or precursors may be added to the medium in a batch or continuous manner, but are not limited thereto.

The pH of the medium may be adjusted by adding compounds, such as ammonium hydroxide, potassium hydroxide, ammonia, phosphoric acid, sulfuric acid, etc., to the medium in an appropriate manner during culturing of the microorganism of the present disclosure. In addition, an anti-foaming agent, such as a fatty acid polyglycol ester, may be used to suppress foam formation during culturing. In addition, an anti-foaming agent, such as a fatty acid polyglycol ester, may be used to suppress foam formation during culturing. In addition, oxygen or oxygen-comprising gas may be injected into the medium to maintain the aerobic state of the medium, or no gas may be injected, or nitrogen, hydrogen, or carbon dioxide gas may be injected to maintain the anaerobic or non-aerobic state, but this is not limited thereto.

Further, the culture medium may comprise metal salts, such as magnesium sulfate or iron sulfate, necessary for growth. Finally, in addition to the above substances, essential growth substances, such as amino acids and vitamins, may be used. Appropriate precursors may also be used in the culture medium. The above-mentioned raw materials may be added to the culture in a batch or continuous type in an appropriate manner during the culture process, but are not limited thereto.

In the present disclosure, the pH of the culture may be adjusted by adding compounds, such as ammonium hydroxide, potassium hydroxide, ammonia, phosphoric acid, sulfuric acid, etc., to the culture in an appropriate manner during culturing of the microorganism. In addition, an anti-foaming agent, such as a fatty acid polyglycol ester, may be used to suppress foam formation during culturing. In addition, oxygen or oxygen-comprising gas may be injected into the culture to maintain the aerobic state of the culture, or no gas may be injected, or nitrogen, hydrogen, or carbon dioxide gas may be injected to maintain the anaerobic or non-aerobic state, but this is not limited thereto.

In the culturing of the present disclosure, the culture temperature may be maintained at 20°C to 35°C, specifically 25°C to 35°C, and the culturing may be continued until the desired amount of a useful material is obtained, and may be carried out for about 10 hours to about 160 hours, about 20 hours to about 130 hours, about 24 hours to about 120 hours, about 36 hours to about 120 hours, about 48 hours to about 120 hours, about 48 hours or more, or about 48 hours, about 72 hours, or about 120 hours, but is not limited thereto.

The L-amino acid produced by culturing of the present disclosure may be released into the medium or may remain in the cells.

In one specific embodiment of the present disclosure, the L-amino acid may be any one or more selected from L-histidine, L-isoleucine, L-tryptophan, and L-lysine, but is not limited thereto.

In one specific embodiment, the method of producing the L-amino acid of the present disclosure may further comprise the steps of preparing the microorganism of the present disclosure, preparing a medium for culturing the strain, or a combination thereof (regardless of the order, in any order), for example, before the culturing step.

The method of producing the L-amino acid of the present disclosure may further comprise the step of recovering the target material, specifically the L-amino acid from the cultured microorganism, a culture of the microorganism, a fermentation product of the microorganism, or the culture medium. The recovering step may be further comprised after the culturing step.

The recovering is to collect the desired L-amino acid using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, etc., HPLC, and a combination thereof may be used, and the desired substance, specifically the L-amino acid, may be recovered from the medium or the microorganism using suitable methods known in the art.

In addition, the method of producing the L-amino acid of the present disclosure may further comprise a purification step. The purification may be performed by using an appropriate method known in the art. In an exemplary embodiment, when the method of producing the L-amino acid of the present disclosure comprises both the recovering step and the purification step, the recovering step and the purification step may be performed continuously or discontinuously regardless of the order, or may be performed simultaneously or integrated into one step, but this is not limited thereto.

With regard to the method of the present disclosure, the variant polypeptide, introduction, and L-amino acid, etc. are as described in other aspects.

Still another aspect of the present disclosure provides a composition for producing the L-amino acid, the composition comprising any one or more selected from the variant polypeptide of the present disclosure; the polynucleotide encoding the variant polypeptide; the vector comprising the same; the microorganism comprising the vector; a culture of the microorganism; and a fermentation product of the microorganism.

The composition of the present disclosure may further comprise any appropriate excipient that is usually used in a composition for producing L-amino acids, and such excipients may comprise, for example, a preserving agent, a wetting agent, a dispersing agent, a suspending agent, a buffering agent, a stabilizing agent, an isotonic agent, etc., but are not limited thereto.

In one specific embodiment, each component present in the composition of the present disclosure may be comprised in a microbiologically effective amount, or in an amount that may be appropriately present in the composition for production.

With regard to the composition of the present disclosure, the variant polypeptide, introduction, and L-amino acid, etc. are as described in other aspects.

Still another aspect of the present disclosure provides use of the microorganism having the improved ability to produce the L-amino acid, into which any one or more selected from the variant polypeptide of the present disclosure; and the polynucleotide encoding the variant polypeptide are introduced, in producing the L-amino acid.

With regard to the use of the present disclosure, the variant polypeptide, introduction, and L-amino acid, etc. are as described in other aspects.

Still another aspect of the present disclosure provides a method of preparing the microorganism having the improved ability to produce the L-amino acid, the method comprising the step of introducing any one or more selected from the variant polypeptide of the present disclosure; and the polynucleotide encoding the variant polypeptide into the microorganism of the genus *Corynebacterium* having the ability to produce the L-amino acid.

Still another aspect of the present disclosure provides a method of increasing the ability to produce the L-amino acid, the method comprising the step of introducing any one or more selected from the variant polypeptide of the present disclosure; and the polynucleotide encoding the variant polypeptide into the microorganism of the genus *Corynebacterium* having the ability to produce the L-amino acid.

With regard to the method of the present disclosure, the variant polypeptide, introduction, and L-amino acid, etc. are as described in other aspects.

### [Detailed Description of Preferred Embodiments]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Construction of Recombinant Vector for Introducing Fructokinase Mutations

It was aimed to prepare a *Corynebacterium glutamicum* mutant strain with improved fructokinase activity. In detail, in order to construct a vector for deletion of Tn1 transposon-encoding gene and insertion of a target gene in *Corynebacterium glutamicum,* genomic DNA of *Corynebacterium glutamicum* ATCC13032 as a template and primer pairs of SEQ ID NO: 9 and SEQ ID NO: 10, and SEQ ID NO: 11 and SEQ ID NO: 12 were used to perform PCR, respectively. PfuUltra^{™} high-fidelity DNA polymerase (Stratagene) was used as a polymerase for the PCR reaction, and the PCR conditions were denaturation at 95°C for 30 seconds; denaturation at 55°C for 30 seconds; and polymerization at 72°C for 1 minute. The denaturation, annealing, and polymerization of the conditions were repeated for 28 cycles. As a result, DNA fragments of 511 bp and 526 bp were obtained, respectively.

**[Table 1]**

| SEQ ID NO. | Name | Sequence |
|---|---|---|
| 9 | Primer | ttcgagctcggtacccCAAATGCTCCAACCGTCCGT |
| 10 | Primer | CGCTGGGATGTTTCTGAACTCATTCCTTCTGCT |
| 11 | Primer | ACAAGAACTGGAATAGGACATCTAATAACCGGGC |
| 12 | Primer | ctctagaggatccccTACACCTCCACCTGCCCAG |

Subsequently, genomic DNA of wild-type *Escherichia coli* ATCC 9637 as a template and a primer pair of SEQ ID NOS: 13 and 14 were used to perform PCR, thereby obtaining a wild-type csck (*E. coli*) gene fragment. Information on the corresponding gene and surrounding nucleotide sequences (Accession Number CP002967.1) was obtained from the National Institutes of Health GenBank (NIH GenBank).

Further, genomic DNA of *Escherichia coli* ATCC 9637 as a template, and a primer pair of SEQ ID NOS: 13 and 15 and a primer pair of SEQ ID NOS: 16 and 14 were used to perform PCR, respectively. A mixture of these obtained PCR fragments as a template and a primer pair of SEQ ID NOs: 13 and 14 were used to perform overlapping PCR, thereby obtaining a *'cscK* (W79C, *E. coli*)' gene fragment. Similarly, genomic DNA of *Escherichia coli* ATCC 9637 as a template and a primer pair of SEQ ID NOS: 13 and 17 and a primer pair of SEQ ID NOS: 18 and 14 were used to perform PCR, respectively, and a mixture of the two obtained fragments as a template and a primer pair of SEQ ID NO: 13 and SEQ ID NO: 14 were used to perform overlapping PCR again, thereby obtaining a *'cscK* (A181V, E.co)' gene fragment. In addition, in order to prepare a combination variant, genomic DNA of *Escherichia coli* ATCC 9637 as a template and the primer pair of SEQ ID NO: 13 and SEQ ID NO: 15, the primer pair of SEQ ID NO: 16 and SEQ ID NO: 17, and the primer pair of SEQ ID NO: 18 and SEQ ID NO: 14 were used to perform PCR, respectively, and a mixture of the three obtained fragments as a template and the primer pair of SEQ ID NO: 13 and SEQ ID NO: 14 were used to perform overlapping PCR again, thereby obtaining a '*cscK*(W79C/A181V, E.co)' gene fragment. PCR was performed in the same manner as the above conditions.

**[Table 2]**

| SEQ ID NO. | Name | Sequence |
|---|---|---|
| 13 | Primer | CCCAACGAAAGGAAACACTCATGTCAGCCAAAGTAT GGGT |
| 14 | Primer | TGCCCGGTTATTAGATGTCCTATTCCAGTTCTTGTC |
| 15 | Primer | ATGTCCGGTGACATTCATCTTGC |
| 16 | Primer | AAGATGAATGTCACCGGACATCC |
| 17 | Primer | ACATCCACCAGTTGTAGCGCCTGCCGCAAA |
| 18 | Primer | AACTGGTGGATGTCGTCAAGCTCTCGGAAG |

Subsequently, to use the CJ7 promoter derived from *Corynebacterium ammoniagenes* (SEQ ID NO: 19, Korean Patent No. 10-0620092), genomic DNA of *Corynebacterium ammoniagenes* as a template and primers of SEQ ID NO: 20 and SEQ ID NO: 21 were used to perform PCR in the same manner as above, thereby obtaining a CJ7 promoter fragment.

**[Table 3]**

| SEQ ID NO. | Name | Sequence |
|---|---|---|
| 20 | Primer | GCAGAAGGAATGAGTTCAGAAACATCCCAGCGCTAC T |
| 21 | Primer | TACTTTGGCTGACATGAGTGTTTCCTTTCGTTGGGT |

Each of the obtained DNA products was purified using a PCR purification kit (QUIAGEN). The purified DNA product and a chromosomal transformation vector pDC24 (SEQ ID NO: 85) digested with the Smal restriction enzyme were cloned using the Gibson assembly method (DG Gibson et al., NATURE METHODS, VOL.6 NO.5, MAY 2009, NEBuilder HiFi DNA Assembly Master Mix), thereby obtaining recombinant plasmids, which were named pDC24△Tn1::cj7_cscK(E.co), pDC24△Tn1::Pcj7_cscK(W79C, E.co), pDC24△Tn1::Pcj7_cscK(A181V, E.co), and pDC24△Tn1::Pcj7_cscK(W79C/A181V, E.co), respectively. Cloning was performed by mixing a Gibson assembly reagent and each gene fragment in a calculated molar number and storing the mixture at 50°C for 1 hour.

### Example 2. Evaluation of Production Ability of L-Amino Acid-Producing Strain Introduced with cscK Mutant Gene

### Example 2-1. Preparation of Histidine-Producing Strain and Evaluation of Histidine-Producing Ability

### Example 2-1-1. Preparation of Histidine-Producing Strain

In order to evaluate the L-histidine-producing ability of the strain into which the cscK mutant gene prepared in Example 1 was introduced, a CA14-0114 strain with enhanced genes in the histidine biosynthetic pathway was first prepared using wild-type *Corynebacterium glutamicum* ATCC13032 as a starting microorganism. In detail, in order to release the feedback inhibition of the HisG protein, which is the first enzyme in the L-histidine biosynthetic pathway, the codon of the *hisG* gene was modified to express a protein (SEQ ID NO: 22) (ACS Synth. Biol., 2014, 3 (1), pp 21-29) in which the amino acids at positions 233 and 235 from the N-terminus of *HisG* were simultaneously substituted from glycine to histidine and from threonine to glutamine, respectively. In addition, the start codon was substituted from GTG to ATG to enhance the activity of the *hisE* gene, which is present in the same operon as *hisG.* Additionally, to strengthen the L-histidine biosynthetic pathway, promoters of the biosynthetic genes *hisN, hisH, hisD, hisA* and *hisB* were replaced with strong promoters, and the corresponding pathway was strengthened by introducing additional copies of the hisE(g1a)G(G233H/T235Q) operon and *hisD* gene.

### Example 2-1-1-1. Preparation of Histidine-Producing Strain with Released Feedback Inhibition

To prepare a histidine-producing strain in which feedback inhibition was released, genomic DNA of *Corynebacterium glutamicum* ATCC13032 as a template and a primer pair of SEQ ID NO: 23 and SEQ ID NO: 24, and a primer pair of SEQ ID NO: 25 and SEQ ID NO: 26 were used to perform PCR. The PCR reaction was performed in the same manner as in Example 1. The two amplified DNA fragments as templates and primers of SEQ ID NO: 23 and SEQ ID NO: 26 were used to perform PCR in the same manner as in Example 1, thereby obtaining a *'hisE(g1a)G(G233H*/*T235Q)'* gene fragment. Further, the upstream region of the *hisE* gene was obtained by performing PCR using chromosomal DNA of ATCC13032 as a template and primers of SEQ ID NOS: 27 and 28.

**[Table 4]**

| SEQ ID NO. | Name | Sequence (5'-> 3') |
|---|---|---|
| 23 | Primer | gaggaggatcaaaacaATGAAGACATTTGAC |
| 24 | Primer | ACACCAAGCTTGATGGATACCTCTACTGCA |
| 25 | Primer | CAGTAGAGGTATCCATCAAGCTTGGTGTC |
| 26 | Primer | ACTCTAGAGGATCCCCCTAGATGCGGGC |
| 27 | Primer | TCGAGCTCGGTACCCACCGAACTCCTGACAGAGT |
| 28 | Primer | acatgaagcgccTCGGTACATTCTTCCACA |
| 30 | Primer | AAGAATGTACCGAggcgcttcatgtcaaca |
| 31 | Primer | CAAATGTCTTCATtgttttgatctcctcca |
| 32 | Primer | ACTGCCTGGTACCACCAGA |
| 33 | Primer | CTGCCTCTCACAAGTTGAAG |

To replace with a strong promoter, a synthetic Pspl13 promoter (SEQ ID NO: 29, Korean Patent No. 10-1783170) as a template and primers of SEQ ID NOS: 30 and 31 were used to perform PCR in the same manner as in Example 1.

After treating the pDC24 vector with the restriction enzyme Smal, the amplified upstream DNA fragment of the *hisE* gene, the Pspl13 promoter, and the *'hisE(g1a)G(G233H*/*T235Q)'* gene fragment were cloned using the Gibson assembly method to obtain a recombinant plasmid, which was named pDC24△Pn_hisEG:: Pspl13_hisE(g1a)G(G233H/T235Q). Gibson cloning was performed in the same manner as in Example 1.

The constructed pDC24△Pn_hisEG:: Pspl13_hisE(g1a)G(G233H/T235Q) vector was transformed into *Corynebacterium glutamicum* ATCC13032 by electroporation, and then through a second crossover, a mutation was introduced into the existing *hisG* gene, thereby obtaining a strain in which feedback inhibition was released and *hisE* activity was enhanced by replacing the start codon of the *hisE* gene. The corresponding genetic manipulation was confirmed by PCR using primers of SEQ ID NO: 32 and SEQ ID NO: 33 which are able to respectively amplify the external regions of the homologous recombination upstream region and downstream region where the corresponding gene was inserted, and genome sequencing. The strain thus obtained was named CJ-HIS1.

### Example 2-1-1-2. Preparation of Histidine-Producing Strain with Enhanced Biosynthetic Pathway through Promoter Replacement

Next, in order to enhance the activities of *hisN, hisH, hisD, hisA,* and *hisB* which are biosynthetic genes, plasmids for replacing the wild-type promoter of each gene with a strong promoter were constructed.

In detail, chromosomal DNA of *Corynebacterium glutamicum* ATCC13032 as a template and primers of SEQ ID NO: 34 and SEQ ID NO: 35, SEQ ID NO: 36 and SEQ ID NO: 37, SEQ ID NO: 38 and SEQ ID NO: 39, SEQ ID NO: 40 and SEQ ID NO: 41, and SEQ ID NO: 42 and SEQ ID NO: 43 were used to perform PCR in the same manner as in Example 1, thereby obtaining the upstream regions of *hisN, hisH, hisD, hisA,* and *hisB* genes, respectively. Further, chromosomal DNA of *Corynebacterium glutamicum* ATCC13032 as a template and primers of SEQ ID NO: 44 and SEQ ID NO: 45, SEQ ID NO: 46 and SEQ ID NO: 47, SEQ ID NO: 48 and SEQ ID NO: 49, SEQ ID NO: 50 and SEQ ID NO: 51, and SEQ ID NO: 52 and SEQ ID NO: 53 were used to obtain the downstream regions of *hisN, hisH, hisD, hisA,* and *hisB* genes, respectively.

To replace the endogenous promoters of *hisN, hisH,* and *hisD* genes with a strong Pcj7 promoter, genomic DNA of *Corynebacterium ammoniagenes* as a template and primers of SEQ ID NO: 54 and SEQ ID NO: 55, SEQ ID NO: 56 and SEQ ID NO: 57, and SEQ ID NO: 58 and SEQ ID NO: 59 were used to perform PCR in the same manner as in Example 1.

Further, to replace the endogenous promoters of the *hisA* and *hisB* genes with a strong Pspl13 promoter, the Pspl13 promoter as a template and primers of SEQ ID NO: 60 and SEQ ID NO: 61, SEQ ID NO: 62 and SEQ ID NO: 63 were used to perform PCR in the same manner as in Example 1.

After treating the pDC24 vector with the restriction enzyme Smal, the amplified upstream DNA fragments of *hisN, hisH,* and *hisD* genes, the Pcj7 promoter fragment, and the downstream DNA fragments of *hisN, hisH,* and *hisD* genes were cloned using the Gibson assembly method, thereby obtaining recombinant plasmids, which were named pDC24△Pn:: cj7_hisN, pDC24ΔPn:: cj7_hisH, and pDC24ΔPn:: cj7_hisD, respectively. In addition, after treating the pDC24 vector with the restriction enzyme Smal, the amplified upstream DNA fragments of *hisA* and *hisB* genes, the Pspl13 promoter fragment, and the downstream DNA fragments of *hisA* and *hisB* genes were cloned using the Gibson assembly method, thereby obtaining recombinant plasmids, which were named pDC24ΔPn:: Pspl13_hisA and pDC24ΔPn:: Pspl13_hisB, respectively. Gibson cloning was performed in the same manner as in Example 1.

The constructed pDC24ΔPn:: cj7_hisN vector was transformed into CJ-HIS1, which was prepared in Example 2-1-1-1, by electroporation. Through a second crossover, the promoter in the existing *hisN* gene was replaced, thereby obtaining a strain in which the corresponding gene was enhanced. The corresponding genetic manipulation was confirmed by PCR using primers of SEQ ID NO: 64 and SEQ ID NO: 65 which are able to respectively amplify the external regions of the homologous recombination upstream region and downstream region where the corresponding gene was inserted, and genome sequencing. The strain thus obtained was named CJ-HIS2.

Next, the constructed pDC24ΔPn:: cj7_hisH vector was transformed into CJ-HIS2 prepared above, by electroporation. Through a second crossover, the promoter in the existing *hisH* gene was replaced, thereby obtaining a strain in which the corresponding gene was enhanced. The corresponding genetic manipulation was confirmed by PCR using primers of SEQ ID NO: 66 and SEQ ID NO: 67 which are able to respectively amplify the external regions of the homologous recombination upstream region and downstream region where the corresponding gene was inserted, and genome sequencing. The strain thus obtained was named CJ-HIS3.

Next, the constructed pDC24△Pn:: cj7_hisD vector was transformed into CJ-HIS3 prepared above, by electroporation. Through a second crossover, the promoter in the existing *hisD* gene was replaced, thereby obtaining a strain in which the corresponding gene was enhanced. The corresponding genetic manipulation was confirmed by PCR using primers of SEQ ID NO: 68 and SEQ ID NO: 69, and genome sequencing. The strain thus obtained was named CJ-HIS4.

Next, the constructed pDC24△Pn:: Pspl13_hisA vector was transformed into CJ-HIS4 prepared above, by electroporation. Through a second crossover, the promoter in the existing *hisA* gene was replaced, thereby obtaining a strain in which the corresponding gene was enhanced. The corresponding genetic manipulation was confirmed by PCR using primers of SEQ ID NO: 70 and SEQ ID NO: 71, and genome sequencing. The strain thus obtained was named CJ-HIS5.

Next, the constructed pDC24△Pn:: Pspl13_hisB vector was transformed into CJ-HIS5 prepared above, by electroporation. Through a second crossover, the promoter in the existing *hisB* gene was replaced, thereby obtaining a strain in which the corresponding gene was enhanced. The corresponding genetic manipulation was confirmed by PCR using primers of SEQ ID NO: 72 and SEQ ID NO: 73, and genome sequencing. The strain thus obtained was named CJ-HIS6.

**[Table 5]**

| | | |
|---|---|---|
| 34 | Primer | TCGAGCTCGGTACCCATTGGTGCTCGGCGC |
| 35 | Primer | tgggatgtttctGTGTTGTTAGTCTAGTG |
| 36 | Primer | TCGAGCTCGGTACCCAACCAAGTTTAGATGCGCC |
| 37 | Primer | gctgggatgtttctGCCGATAGTTTATGTCA |
| 38 | Primer | TCGAGCTCGGTACCCGGTGACAGCTCGCGCCGCAT |
| 39 | Primer | gcgctgggatgtttctGGCGAAAAGTTCTCCC |
| 40 | Primer | TCGAGCTCGGTACCCTTGATGCCTGCATGAAGG |
| 41 | Primer | tgacatgaagcgccGAATATTGATCCTATCT |
| 42 | Primer | TTCGAGCTCGGTACCCACCTTCAGCAACCACTC |
| 43 | Primer | tgacatgaagcgccGAAAAATTCTTCTCT |
| 44 | Primer | aaaggaaacactcATGAGCAAATATGCAGACG |
| 45 | Primer | CTAGAGGATCCCCCAGCCGGAGAGGGA |
| 46 | Primer | aaaggaaacactcATGACCAAAACTGTCGC |
| 47 | Primer | CTAGAGGATCCCCACCTCTGGAGGCGTGGTC |
| 48 | Primer | cgaaaggaaacactcATGTTGAATGTCACTGACC |
| 49 | Primer | CTAGAGGATCCCCCCGTGCTCAGCCTGAGGAG |
| 50 | Primer | ggagatcaaaacaATGACCTTCACTATTCTTCC |
| 51 | Primer | CTAGAGGATCCCCACGAAACGTGCACAACCTT |
| 52 | Primer | gagatcaaaacaATGACTGTCGCACCA |
| 53 | Primer | CTAGAGGATCCCCGGGTCGCGGCCGTAGTGGC |
| 54 | Primer | ACTAGACTAACAACACagaaacatcccagcgc |
| 55 | Primer | TCTGCATATTTGCTCATgagtgtttccttt |
| 56 | Primer | ACATAAACTATCGGCagaaacatcccagcgcta |
| 57 | Primer | GACAGTTTTGGTCATgagtgtttcctttcg |
| 58 | Primer | GAGAACTTTTCGCCagaaacatcccagcgct |
| 59 | Primer | AGTGACATTCAACATgagtgtttcctttcg |
| 60 | Primer | AGGATCAATATTCggcgcttcatgtcaac |
| 61 | Primer | GAATAGTGAAGGTCATtgttttgatctcct |
| 62 | Primer | GAGAAGAATTTTTCggcgcttcatgtcaa |
| 63 | Primer | TGGTGCGACAGTCATtgttttgatctcct |
| 64 | Primer | GAGCATGCATCAAAG |
| 65 | Primer | AGAAATTTGATCCTTATAA |
| 66 | Primer | TTGAGAGATGCTTATCG |
| 67 | Primer | CACTTCAGTGCGGATTCCAA |
| 68 | Primer | AGCGGGTTTAATTCAGG |
| 69 | Primer | GTGGGTAAGGGTTTTCGT |
| 70 | Primer | CACGAAAATGATCGTTTTG |
| 71 | Primer | TATGGGATTCGATGGCCA |
| 72 | Primer | TGTGGGAATCGCTGGGCAC |
| 73 | Primer | CGGTCGCCCGCATCTG |

### Example 2-1-1-3. Preparation of Histidine-Producing Strain with Enhanced Biosynthetic Pathway through Additional Gene Insertion

Next, NCgl1021, which is known as a gene encoding a transposon in *Corynebacterium glutamicum,* was used as an insertion site to additionally insert the hisE(g1a)G(G233H/T235Q) operon and *hisD* gene. In detail, to construct a vector for NCgl1021 (SEQ ID NO: 74) deletion and target gene insertion, PCR was performed in the same manner as in Example 1 using the chromosome of ATCC13032 as a template and primer pairs of SEQ ID NO: 75 and SEQ ID NO: 76, and SEQ ID NO: 77 and SEQ ID NO: 78 to amplify the left homology arm region of NCgl1021 and the right homology arm region of NCgl1021. PCR was performed in the same manner as in Example 1 using pDC24 △Pn:: Pspl13_ hisE(g1a)G(G233H/T235Q), which was the vector constructed in Example 2-1-1-1, as a template and primers of SEQ ID NO: 79 and SEQ ID NO: 80 to obtain a *'Pspl13_ hisE(g1a)G(G233H*/*T235Q)'* gene fragment.

In addition, PCR was performed in the same manner as in Example 1 using pDC24 △Pn:: cj7_hisD, which was the vector constructed in Example 2-1-1-2, as a template and primers of SEQ ID NO: 81 and SEQ ID NO: 82 to obtain a *'Pcj7_hisD'* gene fragment.

After treating the pDC24 vector with the restriction enzyme Smal, the amplified left homology arm and right homology arm regions of NCgl1021, 'Pspl13_ hisE(g1a)G(G233H/T235Q)' and *'Pcj7_hisD'* gene fragment were cloned using the Gibson assembly method to obtain a recombinant plasmid, which was named 'pDC24△NCgl1021:: Pspl13_ hisE(g1a)G(G233H/T235Q)-Pcj7_hisD'. Gibson cloning was performed in the same manner as in Example 1. The constructed 'pDC24△NCgl1021:: Pspl13_ hisE(g1a)G(G233H/T235Q)-Pcj7_hisD' vector was transformed into CJ-HIS6 prepared in Example 2-1-1-2 by electroporation, and then through a second crossover, a strain was obtained, in which the histidine biosynthetic pathway was enhanced by additional gene insertion. The corresponding genetic manipulation was confirmed by PCR using primers of SEQ ID NO: 83 and SEQ ID NO: 84 which are able to respectively amplify the external regions of the homologous recombination upstream region and downstream region where the corresponding gene was inserted, and genome sequencing. The strain thus obtained was named CA14-0114.

**[Table 6]**

| SEQ ID NO. | Name | Sequence (5'-> 3') |
|---|---|---|
| 75 | Primer | TTCGAGCTCGGTACCCATGAAGTCTACCGGC |
| 76 | Primer | gacatgaagcgccGACATCTAATAACCGGG |
| 77 | Primer | CCGACGAGGCCTAAGAACTCATTCCTTCTGCT |
| 78 | Primer | CTCTAGAGGATCCCCTTAGAGTGCATTGATC |
| 79 | Primer | CCGGTTATTAGATGTCggcgcttcatgtca |
| 80 | Primer | ggatgtttctCTAGATGCGGGCGAT |
| 81 | Primer | GCCCGCATCTAGagaaacatcccagcgct |
| 82 | Primer | AGAAGGAATGAGTTCTTAGGCCTCGTCGG |
| 83 | Primer | CTTTCAGCTTTCCCTCCCG |
| 84 | Primer | GCTGTACTTTTAGTACA |

### Example 2-1-2. Preparation of Transformant Strain by Introduction of csck (E.co) Mutant Gene into Histidine-Producing Strain

The recombinant vectors pDC24△Tn1::Pcj7_cscK(E.co), pDC24△Tn1::Pcj7_cscK(W79C, E.co), pDC24△Tn1::Pcj7_cscK(A181V, E.co) and pDC24△Tn1::Pcj7_cscK(W79C/A181V, E.co), constructed in Example 1, were each transformed into the histidine-producing strain CA14-0114 prepared in Example 2-1-1 by an electroporation. Then, transformant strains were obtained on a selective medium comprising 25 mg/L of kanamycin, which were named CA14-0114△Tn1:: Pcj7_cscK(E.co), CA14-0114ΔTn1:: Pcj7_cscK(W79C, E.co), CA14-0114△Tn1:: Pcj7_cscK(A181V, E.co), and CA14-0114△Tn1::Pcj7_cscK(W79C/A181V, E.co), respectively.

### Example 2-1-3. Evaluation of Histidine-Producing Ability

In order to examine the L-histidine-producing ability of the strains prepared in Example 2-1-2, the strains were cultured in the following manner for evaluation. Each of the strains was inoculated into a 250-mL corner-baffled flask comprising 25 mL of the following seed medium, and cultured with shaking at 200 rpm at 30°C for 20 hours. Next, 1 mL of the seed culture solution was inoculated into a 250-mL corner-baffled flask comprising 25 mL of a production medium, and cultured with shaking at 200 rpm at 30°C for 48 hours.

### <Seed Medium (pH 7.0)>

5% Glucose, 1% Bacto-Peptone, 0.25% Sodium Chloride, 1% Yeast Extract, 0.4% Urea (based on 1 liter of distilled water)

### <Production Medium (pH 7.0)>

6% Raw sugar, 2% Ammonium sulfate, 0.1% Potassium Phosphate Monobasic, 0.05% Magnesium Sulfate Heptahydrate, 2.0% Corn Steep Liquor (CSL), 200 µg/L Biotin, 30 g/L Calcium Carbonate (based on 1 liter of distilled water)

**[Table 7]**

| Comparison of L-histidine-producing ability of L-histidine-producing strains derived from *Corynebacterium glutamicum* ATCC 13032 (48H) | |
|---|---|
| Name of strain | L-histidine concentration (g/L) |
| CA14-0114 | 4.3 |
| CA14-0114ΔTn1:: Pcj7-cscK(WT, E.co) | 4.5 |
| CA14-0114ΔTn1:: Pcj7-cscK(W79C, E.co) | 4.9 |
| CA14-0114ΔTn1:: Pcj7-cscK(A181V, E.co) | 4.6 |
| CA14-0114ΔTn1:: Pcj7-cscK(W79C/A181V, E.co) | 4.9 |

As shown in the table above, it was confirmed that introduction of the wild-type *csck* gene derived from *Escherichia coli* increased histidine production by about 4.6%, as compared to the parent strain, CA14-0114. Further, it was confirmed that introduction of the *cscK* variant gene increased histidine production by about 2.2% to about 8.8%, as compared to CA14-0114ΔTn1:: Pcj7-cscK(WT, E.co), into which the wild-type *cscK* gene was introduced. These results indicate that the *csck* gene mutation enhances the fructokinase activity, thereby enhancing the strain's L-histidine-producing ability.

### Example 2-2. Preparation of Isoleucine-Producing Strain Introduced with CscK Mutant and Evaluation of Isoleucine-Producing Ability

### Example 2-2-1. Preparation of Transformant Strain by Introduction of csck(E.co) Mutant Gene into Isoleucine-Producing Strain

To determine whether the introduction of the fructokinase variant gene derived from *Escherichia coli* increases isoleucine production in *Corynebacterium glutamicum* strains having the ability to produce L-isoleucine, the recombinant vectors pDC24△Tn1::Pcj7_cscK(E.co), pDC24ΔTn1:: Pcj7_cscK(W79C, E.co), pDC24ΔTn1:: Pcj7_cscK(A181V, E.co), and pDC24ΔTn1:: Pcj7_cscK(W79C/A181V, E.co), constructed in Example 1, were each transformed into an isoleucine-producing strain KCCM12739P (Korean Patent No. 10-2363913) by an electroporation. Transformant strains were obtained on a selective medium comprising 25 mg/L of kanamycin, which were named KCCM12739PΔTn1:: Pcj7_cscK(E.co), KCCM12739PΔTn1:: Pcj7_cscK(W79C, E.co), KCCM12739PΔTn1:: Pcj7_cscK(A181V, E.co), and KCCM12739PΔTn1:: Pcj7_cscK(W79C/A181V, E.co), respectively.

### Example 2-2-2. Evaluation of Isoleucine-Producing Ability

In order to examine the L-isoleucine-producing ability of the strains prepared in Example 2-2-1, the strains were cultured in the following manner for evaluation. The parent strain and the mutant strains were each inoculated into a 250-mL corner-baffled flask comprising 25 mL of an isoleucine production medium, and cultured with shaking at 200 rpm at 32°C for 60 hours.

### <Production Medium (pH 7.2)>

10% Glucose, 0.2% Yeast Extract, 1.6% Ammonium Sulfate, 0.1% Potassium Phosphate Monobasic, 0.1% Magnesium Sulfate Heptahydrate, 10 mg/l Iron Sulfate Heptahydrate, 10 mg/l Manganese Sulfate Monohydrate, 200 µg/l Biotin (based on 1 L of distilled water)

**[Table 8]**

| Comparison of L-isoleucine-producing ability of L-isoleucine-producing strains derived from *Corynebacterium glutamicum* ATCC 13032 (60H) | |
|---|---|
| Name of strain | L-isoleucine concentration (g/L) |
| KCCM12739P | 2.1 |
| KCCM12739PΔTn1:: Pcj7-cscK(WT, E.co) | 2.3 |
| KCCM12739PΔTn1:: Pcj7-cscK(W79C, E.co) | 2.9 |
| KCCM12739PΔTn1:: Pcj7-cscK(A181V, E.co) | 2.5 |
| KCCM12739P△Tn1:: Pcj7-cscK(W79C/A181V, E.co) | 3.0 |

As shown in the table above, it was confirmed that introduction of the wild-type *csck* gene derived from *Escherichia coli* increased isoleucine production by about 9.5%, as compared to the parent strain, KCCM12739P. Further, it was confirmed that introduction of the csc*K* variant gene increased isoleucine production by about 8.6% to about 30.4%, as compared to KCCM12739PΔTn1:: Pcj7-cscK(WT, E.co), into which the wild-type csc*K* gene was introduced. These results indicate that the *csck* gene mutation enhances the fructokinase activity, thereby enhancing the strain's L-isoleucine-producing ability.

### Example 2-3. Preparation of Tryptophan-Producing Strain Introduced with CscK Mutant and Evaluation of Tryptophan-Producing Ability

### Example 2-3-1. Preparation of Transformant Strain by Introduction of csck(E.co) Mutant Gene into Tryptophan-Producing Strain

To determine whether the introduction of the fructokinase variant gene derived from *Escherichia coli* increases tryptophan production in *Corynebacterium glutamicum* strains having the ability to produce L-tryptophan, the recombinant vectors pDC24ΔTn1:: Pcj7_cscK(E.co), pDC24ΔTn1:: Pcj7_cscK(W79C, E.co), pDC24ΔTn1:: Pcj7_cscK(A181V, E.co), and pDC24ΔTn1:: Pcj7_cscK(W79C/A181V, E.co), constructed in Example 1, were each transformed into a tryptophan-producing strain CA04-8405 (Korean Patent No. 10-1968317) by an electroporation. Then, transformant strains were obtained on a selective medium comprising 25 mg/L of kanamycin, which were named CA04-8405△Tn1::Pcj7_cscK(E.co), CA04-8405△Tn1:: Pcj7_cscK(W79C, E.co), CA04-8405△Tn1::Pcj7_cscK(A181V, E.co), and CA04-8405△Tn1::Pcj7_cscK(W79C/A181V, E.co), respectively.

### Example 2-3-2. Evaluation of Tryptophan-Producing Ability

In order to examine the L-tryptophan-producing ability of the strains prepared in Example 2-3-1, the strains were cultured in the following manner for evaluation. Each of the strains was inoculated into a 250-mL corner-baffled flask comprising 25 mL of a seed medium, and cultured with shaking at 200 rpm at 30°C for 20 hours. After culturing, a new 250-mL corner-baffled flask comprising 25 mL of a production medium was prepared for each strain, and 1 mL of the above seed culture solution was inoculated thereto, and cultured with shaking at 200 rpm at 30°C for 24 hours.

### <Seed Medium (pH 7.0)>

20 g of Glucose, 10 g of Peptone, 5 g of Yeast Extract, 1.5 g of Urea, 4 g of KH₂PO₄, 8 g of K₂HPO₄, 0.5 g of MgSO₄ 7H₂O, 100 µg of Biotin, 1000 µg of Thiamine HCl, 2000 µg of Calcium Pantothenate, 2000 µg of Nicotinamide (based on 1 L of distilled water)

### <Production Medium (pH 7.0)>

30 g of Glucose, 15 g of (NH₄)₂SO₄, 1.2 g of MgSO₄ 7H₂O, 1 g of KH₂PO₄, 5 g of Yeast Extract, 900 µg of Biotin, 4500 µg of Thiamine HCl, 4500 µg of Calcium Pantothenate, 30 g of CaCO₃ (based on 1 L of distilled water)

**[Table 9]**

| Comparison of L-tryptophan-producing ability of L-tryptophan-producing strains derived from *Corynebacterium glutamicum* ATCC 13869 (24H) | |
|---|---|
| Name of strain | L-tryptophan concentration (g/L) |
| CA04-8405 | 1.5 |
| CA04-8405 ΔTn1:: Pcj7-cscK(WT, E.co) | 1.6 |
| CA04-8405 ΔTn1:: Pcj7-cscK(W79C, E.co) | 1.9 |
| CA04-8405 ΔTn1:: Pcj7-cscK(A181V, E.co) | 1.8 |
| CA04-8405 ΔTn1:: Pcj7-cscK(W79C/A181V, E.co) | 2.0 |

As shown in the table above, it was confirmed that introduction of the wild-type *csck* gene derived from *Escherichia coli* increased tryptophan production, as compared to the parent strain, CA04-8405. Further, it was confirmed that introduction of the csc*K* variant gene increased tryptophan production by about 12.5% to about 25%, as compared to CA04-8405 ΔTn1:: Pcj7-cscK(WT, E.co), into which the wild-type csc*K* gene was introduced. These results indicate that the *csck* gene mutation enhances the fructokinase activity, thereby enhancing the strain's L-tryptophan-producing ability.

### Example 2-4. Preparation of Lysine-Producing Strain Introduced with CscK Mutant and Evaluation of Lysine-Producing Ability

### Example 2-4-1. Preparation of Transformant Strain by Introduction of csck(E.co) Mutant Gene into Lysine-Producing Strain

To determine whether the introduction of the fructokinase variant gene derived from *Escherichia coli* increases lysine production in *Corynebacterium glutamicum* strains having the ability to produce L-lysine, the recombinant vectors pDC24△Tn1:: Pcj7_cscK(E.co), pDC24△Tn1:: Pcj7_cscK(W79C, E.co), pDC24△Tn1:: Pcj7_cscK(A181V, E.co) and pDC24△Tn1:: Pcj7_cscK(W79C/A181V, E.co), constructed in Example 1, were each transformed into a lysine-producing strain KCCM11016P (Korean Patent No. 10-0159812) by an electroporation. Then, transformant strains were obtained on a selective medium comprising 25 mg/L of kanamycin, which were named KCCM11016P△Tn1:: Pcj7_cscK(E.co), KCCM11016P△Tn1:: Pcj7_cscK(W79C, E.co), KCCM11016P△Tn1:: Pcj7_cscK(A181V, E.co), and KCCM11016P△Tn1:: Pcj7_cscK(W79C/A181V, E.co), respectively.

### Example 2-4-2. Evaluation of Lysine-Producing Ability

In order to examine the L-lysine-producing ability of the strains prepared in Example 2-4-1, the strains were cultured in the following manner for evaluation. Each of the strains was inoculated into a 250-mL corner-baffled flask comprising 25 mL of a seed medium, and cultured with shaking at 200 rpm at 37°C for 20 hours. Each strain was inoculated into a 250-mL corner-baffled flask comprising 25 mL of a production medium, and cultured with shaking at 200 rpm at 37°C for 48 hours.

### <Seed Medium (pH 7.0)>

20g of Glucose, 10 g of Peptone, 5 g of Yeast Extract, 1.5 g of Urea, 4 g of KH₂PO₄, 8 g of K₂HPO₄, 0.5 g of MgSO₄ 7H₂O, 0.1 mg of Biotin, 1 mg of Thiamine HCl, 2 mg of Calcium Pantothenate, 2 mg of Nicotinamide (based on 1 L of distilled water)

### <Production Medium (pH 7.0)>

50 g of Glucose, 40 g of (NH₄)₂SO₄, 5 g of Corn Steep Solids, 10 g of BM, 1 g of KH₂PO₄, 0.5 g of MgSO₄ 7H₂O, 100 µg of Biotin, 1000 µg of Thiamine HCl, 2000 µg of Calcium Pantothenate, 3000 µg of nicotinamide, 30 g of CaCO₃ (based on 1 L of distilled water)

**[Table 10]**

| Comparison of L-lysine-producing ability of L-lysine-producing strains derived from *Corynebacterium glutamicum* ATCC 13869 (48H) | |
|---|---|
| Name of strain | L-Ivsine concentration (g/L) |
| KCCM11016P | 9.45 |
| KCCM11016P △Tn1:: Pcj7-cscK(WT, E.co) | 10.4 |
| KCCM11016P △Tn1:: Pcj7-cscK(W79C, E.co) | 11.6 |
| KCCM11016P △Tn1:: Pcj7-cscK(A181V, E.co) | 10.8 |
| KCCM11016P △Tn1:: Pcj7-cscK(W79C/A181V, E.co) | 12.1 |

As shown in the table above, it was confirmed that introduction of the wild-type *csck* gene derived from *Escherichia coli* increased lysine production, as compared to the parent strain, KCCM11016P. Further, it was confirmed that introduction of the *cscK* variant gene increased lysine production by about 3.8% to about 16%, as compared to KCCM11016P ΔTn1:: Pcj7-cscK(WT, E.co), into which the wild-type *cscK* gene was introduced. These results indicate that the *csck* gene mutation enhances the fructokinase activity, thereby enhancing the strain's L-lysine-producing ability.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A fructokinase variant polypeptide, wherein an amino acid corresponding to position 79 or position 181 of SEQ ID NO: 1 is substituted with a different amino acid.

2. The variant polypeptide of claim 1, wherein the variant polypeptide has a substitution of the amino acid corresponding to position 79 of SEQ ID NO: 1 with cysteine or a substitution of the amino acid corresponding to position 181 of SEQ ID NO: 1 with valine, or a combination thereof.

3. The variant polypeptide of claim 1, wherein the amino acid sequence of the variant polypeptide has 90% or more identity to the amino acid sequence of SEQ ID NO: 1.

4. The variant polypeptide of claim 1, wherein the variant polypeptide consists of an amino acid sequence of SEQ ID NO: 3, SEQ ID NO: 5, or SEQ ID NO: 7.

5. A polynucleotide encoding the variant polypeptide of any one of claims 1 to 4.

6. A microorganism comprising any one or more selected from the variant polypeptide of any one of claims 1 to 4; and a polynucleotide encoding the variant polypeptide.

7. The microorganism of claim 6, wherein the microorganism is a microorganism of the genus *Corynebacterium.*

8. The microorganism of claim 7, wherein the microorganism of the genus *Corynebacterium* is *Corynebacterium glutamicum.*

9. The microorganism of claim 6, wherein the microorganism has an increased ability to produce an L-amino acid, as compared to a microorganism comprising the polypeptide of SEQ ID NO: 1 or a polynucleotide encoding the same.

10. The microorganism of claim 9, wherein the L-amino acid is any one or more selected from L-histidine, L-isoleucine, L-tryptophan, and L-lysine.

11. A method of producing an L-amino acid, the method comprising the step of culturing the microorganism of claim 6 in a medium.

12. The method of claim 11, further comprising the step of recovering the L-amino acid from any one or more selected from the cultured microorganism; a culture of the microorganism; a fermentation product of the microorganism; and the culture medium.

13. The method of claim 12, wherein the L-amino acid is any one or more selected from L-histidine, L-isoleucine, L-tryptophan, and L-lysine.

14. Use of the variant polypeptide of any one of claims 1 to 4; or a microorganism comprising the variant polypeptide or a polynucleotide encoding the variant polypeptide in producing an L-amino acid.
